# EUROPÄISCHE PATENTSCHRIFT

(11) **EP 0 429 029 B1**
(45) Veröffentlichungstag und Bekanntmachung des Hinweises auf die Patenterteilung: **25.05.1994**
(21) Anmeldenummer: 90122005.3
(22) Anmeldetag: 17.11.1990
(51) Int. Cl.: A61F 2/52

(54) **Verfahren zur Herstellung einer Brustprothese**
Method for producing a breast prosthesis
Procédé pour la fabrication d'une prothèse mammaire

(30) Priorität: 17.11.1989 DE 3938328
(43) Veröffentlichungstag der Anmeldung: 29.05.1991
(73) Patentinhaber: DEKUMED Gesellschaft für Kunststoff- und Medizintechnik mbH, D-83233 Bernau (DE)
(72) Erfinder: Degler, Peter, Dipl.-Ing. (FH), W-8201 Wildenwart (DE); Kramer, Bernhard, W-8215 Marquartstein (DE)
(74) Vertreter: Patentanwälte Dipl.-Ing. R. Splanemann Dr. B. Reitzner Dipl.-Ing. K. Baronetzky

(56) Entgegenhaltungen:
- DE-A- 2 605 148
- DE-A- 3 336 279
- DE-A- 3 416 240
- US-A- 4 401 492

## Beschreibung

Brustprothesen werden in großem Umfang nach der operativen Entfernung von Mammakarzinomen eingesetzt. Sie werden gewöhnlich aus einer gelartig aushärtenden Kunststoffmasse, insbesondere aus einer Siliconkunstharzmasse, hergestellt, wobei die Außenfläche der Prothese der Brustform nachgebildet ist. Die Brustprothesen können entweder als Vollprothesen oder aus Hohlprothesen ausgebildet sein, wobei im letzteren Fall die dem Körper zugewandte Seite schalenförmig vertieft ist.

Man unterscheidet zwischen folienfreien und mit Folien ummantelten Brustprothesen. Die folienfreien Brustprothesen, die nur einen geringen Prozentsatz der auf dem Markt befindlichen Prothesen ausmachen, haben den Nachteil, daß sie klebrig sind, da die Siliconkunstharzmasse häufig nicht vollständig vernetzt ist, wobei unvernetztes Siliconöl austritt.

Um diesem Nachteil zu begegnen, hat man derartige Brustprothesen bereits mit thermoplastischen Kunststoff-Folien, insbesondere aus Polyurethan, ummantelt. Im allgemeinen werden derartige Brustprothesen dadurch hergestellt, daß man eine noch nicht vernetzte Siliconkunstharzmasse Zusammen mit dem Vernetzer und einem Katalysator zwischen zwei flachliegende, die Prothesehülle bildende Folien einfüllt, die am Prothesenrand bis auf eine Einfüllöffnung miteinander verschweißt sind. Die Folien werden im Bereich des geschweißten Randes auf dem Rand einer der Brustform entsprechenden Aushöhlung einer Matrize fixiert. Die Siliconharzmasse wird so lange eingefüllt, bis die Folien gegen die Wandungen des Matrizenhohlraumes gedrückt werden, worauf die Folienränder auch im Bereich der Einfüllöffnung miteinander verschweißt werden und die in der Matrize befindliche Masse bei erhöhten Temperaturen vernetzt und hierbei zu einer gelartigen Masse ausgehärtet wird.

Brustprothesen dieses Typs sind beispielsweise in den deutschen Offenlegungsschriften DE-A-27 01 627, 27 37 321 und 29 02 373 beschrieben.

Das Verschweißen der Folienränder bereitet jedoch insbesondere im Bereich der Einfüllöffnungen Schwierigkeiten, wenn sich zwischen den Folien noch Reste der eingespritzten Siliconharzmasse befinden. Diese Reste verhindern eine einwandfreie Verschweißung der Folienränder, so daß die Schweißnaht leicht aufreißt und die Siliconharzmasse sowohl während des Aushärtungsvorgangs als auch im ausgehärteten Zustand leicht austritt, auch wenn nur ein geringer Druck auf die Brustprothese ausgeübt wird. Außerdem müssen die Matrizen bei jedem Schweiß-und Vernetzungsvorgang auf eine verhältnismäßig hohe Temperatur (etwa 130°C) erhitzt und dazwischen immer wieder abgekühlt werden, was sehr zeitraubend und kostenaufwendig ist.

Ein weiterer Nachteil des Schweißverfahrens besteht darin, daß die an den beiden Folien anliegenden Schweißelektroden sehr genau aneinander angepaßt werden müssen, um eine haltbare Schweißnaht zu erzielen. Dies ist praktisch nur möglich, wenn die Auflageflächen genau plan sind. Die auf diese Weise erhaltenen Brustprothesen sind deshalb an der an den Körper anliegenden Seite plan, so daß beim Anlegen der Brustprothese eine störende Faltenbildung auftritt.

Außerdem haben die bekannten Brustprothesen infolge ihrer naturgemäß vorgegebenen Weichheit nur eine geringe Formhaltigkeit, so daß sie sich schon aufgrund ihres Gewichts verformen, wenn ihre Abmessungen nicht genau den Abmessungen des Büstenhalters entsprechen.

Um diese Nachteile zu vermeiden, schlägt die DE-C-33 36 279 eine Brustprothese vor, die aus einer gelartig ausgehärteten Kunstharzmasse besteht, die zwischen zwei miteinander verbundenen thermoplastischen Folien eingeschlossen ist und die aus zwei Schichten unterschiedlicher Festigkeit aufgebaut ist; diese Brustprothese ist dadurch gekennzeichnet, daß die dem menschlichen Körper zugewandte Schicht mit höherer Festigkeit aus kondensationsvernetztem Siliconharz und diesem dem menschlichen Körper abgewandte Schicht mit geringerer Festigkeit aus additionsvernetztem Siliconharz aufgebaut sind und daß die Innenseiten der thermoplastischen Folien zumindest in den Randbereichen mit einer gegenüber dem kondensationsvernetzten Siliconharz wirksamen Haftvermittlerschicht versehen sind.

Nach der in der DE-A-34 16 240 beschriebenen Verbesserung dieser Prothese soll die Vernetzungstemperatur im unteren Teil des plastischen Bereichs der Folie liegen.

Bei der Herstellung dieser Brustprothese ist keine Einfüllöffnung für die aushärtbare Kunstharzmasse erforderlich. Es ist jedoch die Anwendung einer Haftvermittlerschicht erforderlich, da die Menge der Kunstharzmasse zur Herstellung der dem Körper zugewandten Schicht nicht genau dosiert werden kann und so bemessen werden muß, daß die Kunstharzmasse zwangsläufig zwischen die äußeren Randbereiche der Folien gelangt. Ferner wurde bei der Herstellung dieser Prothesen häufig eine Faltenbildung beobachtet.

Die Erfindung bezweckt eine Verbesserung derartiger Brustprothesen im Sinne einer Vereinfachung des Herstellungsverfahrens. Insbesondere soll die Füllung der Brustprothese mit der härtbaren Kunstharzmasse bei Aufrechterhaltung einer konstanten, dem Körper zugewandten schalenförmigen Vertiefung, verbessert werden und, schließlich soll eine Verschiebung der beiden Folien während der Herstellung und damit eine Faltenbildung vermieden werden.

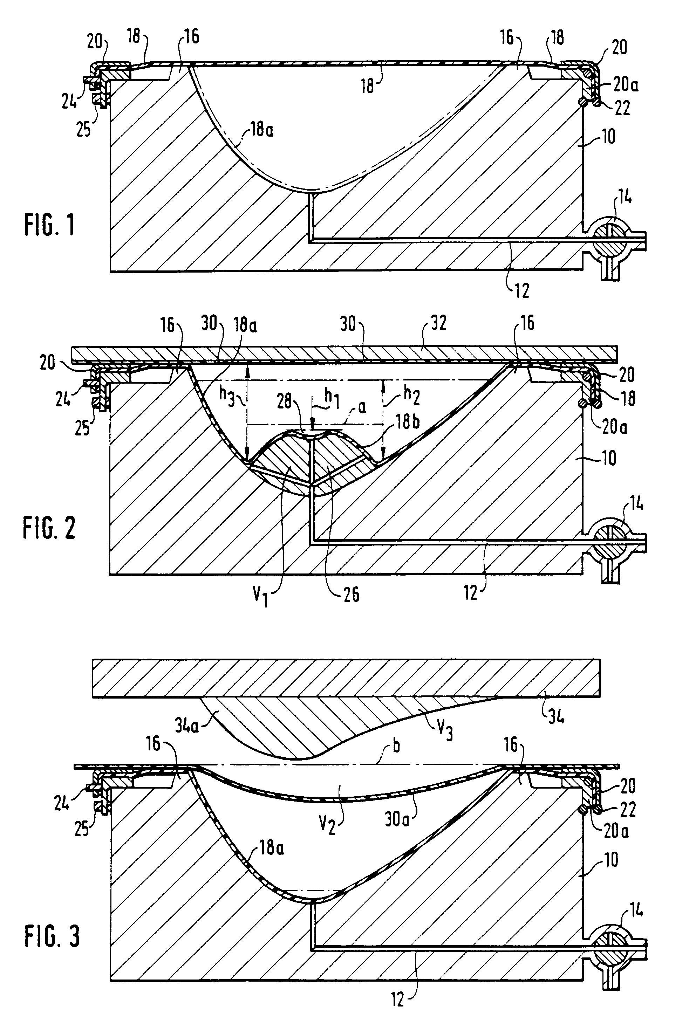

Die Erfindung betrifft somit ein Verfahren zur Herstellung einer Brustprothese
(a) aus einer gelartig härtbaren Kunstharzmasse, die zwischen zwei thermoplastischen Folien eingeschlossen ist,
(b) mit einer einseitigen schalenförmigen Vertiefung,
(c) unter Verwendung einer erwärmten, eine Matrize und eine Patrize enthaltenden Form;
welches folgende Schritte umfaßt:
1) die der schalenförmigen Vertiefung abgewandte, gespannte erste Folie (18) wird auf die Matrize (10) gelegt, erwärmt und durch Tiefziehen dauerhaft den Konturen der Matrize (10) angepaßt (Fig. 1);
2) die tiefgezogene erste Folie (18) wird aus der Matrize (10) entfernt;
3) in die Matrize (10) wird ein Einlagekörper (26) eingesetzt, dessen Volumen (V1) und freie Fläche dem Volumen und der Fläche der schalenförmigen Vertiefung (V2) entsprechen;
4) die tiefgezogene erste Folie (18) wird an den Einlagekörper (26) in der Matrize (10) angelegt (Fig. 2);
5) die Matrize (10) wird bis zu ihrem Rand mit der Kunstharzmasse gefüllt;
6) die Kunstharzmasse wird mit einer zweiten Folie (30) abgedeckt und über deren gesamten Rand (16) mit der ersten Folie (18) verschweißt;
7) der Einlagekörper (26) wird entfernt;
8) die zweite Folie (30) wird mit Hilfe der Patrize (34a), deren Form der Form der schalenförmigen Vertiefung (V2) entspricht, dauerhaft den Konturen der Patrize (34a) anpaßt (Fig. 3);
9) die Kunstharzmasse wird ausgehärtet.

Unter der "freien" Fläche des Einlagekörpers versteht man die Fläche, die nicht mit der Wandung der Matrize in Berührung steht.

Die DE-A-21 26 587 betrifft ein Verfahren zur Herstellung von mehrschichtigen Gegenständen aus Schaumkunststoff, wobei Brustprothesen nicht erwähnt sind. Das DE-U-83 08 257 und die DE-A-28 19 968 beziehen sich zwar auf Brustprothesen; es fehlen jedoch Hinweise auf einen Einlagekörper. Die DE-B-26 50 489 betrifft die Herstellung eines luftgefüllten Innenraums in einer Brustprothese, d.h. es fehlt wiederum jeglicher Hinweis auf einen Einlagekörper.

Bei dem erfindungsgemäßen Verfahren wird also die vorzugsweise in einem Spannrahmen fixierte gespannte erste Folie dauerhaft den Konturen der Matrize angepaßt, bevor die Kunstharzmasse eingefüllt wird. Auf diese Weise wird eine Verschiebung der Folie während der folgenden Verfahrensschritte und damit eine Faltenbildung vermieden. Die erste Folie wird vorzugsweise durch Erwärmen von außen (z.B. durch Strahlung oder durch Warmluft) auf die Erweichungstemperatur erhitzt, während man die Matrize auf einer Temperatur zwischen der Erweichungstemperatur der Folie und der Aushärtungstemperatur der Kunstharzmasse hält. Auf diese Weise kann immer eine konstante Fertigungstemperatur eingehalten werden. Nach dem Stand der Technik erfolgt die endgültige Formgebung der ersten Folie erst am Ende des Aushärtungsvorganges, da die Erweichungstemperatur der Folie höher als die Aushärtungstemperatur der Kunstharzmasse ist. Durch die dauerhafte Verformung der ersten Folie wird ferner erreicht, daß sich diese während der folgenden Verfahrensschritte nicht mehr verschieben kann.

Dieser Effekt wird durch die Verwendung des Einlagekörpers verstärkt, dessen Volumen und freie Fläche dem Volumen und der Fläche der dem Körper zugewandten schalenförmigen Vertiefung entsprechen. Auf diese Weise gibt es nach dem Verschweißen der Folienränder keine Faltenbildung mehr. Es sei betont, daß es nicht ausreicht, wenn der Einlagekörper nur das gleiche Volumen wie die schalenförmige Vertiefung hat, nicht aber die gleiche freie Oberfläche. In einem solchen Fall würde sich die Faltenbildung nicht ausschließen lassen. Durch den Einlagekörper erhält die tiefgezogene erste Folie lediglich eine "Eindellung"; nach dem Entfernen des Einlagekörpers geht die Folie wieder in die Form zurück, die sie durch das Tiefziehen erhalten hat. Damit sich die tiefgezogene Folie nicht mehr verformen kann, setzt man den Einsatzkörper vorzugsweise bei einer Temperatur unterhalb der Erweichungstemperatur der Matrize ein. Die Temperatur des Einlagekörpers kann hierbei noch unterhalb der Temperatur der Matrize liegen. Die tiefgezogene Folie wird in jedem Fall aber dicht an den Einlagekörper in der Matrize angelegt, damit das effektive Volumen des von der ersten Folie umhüllten Einlagekörpers dem Volumen der dem Körper zugewandten schalenförmigen Vertiefung entspricht.

Anschließend wird die Matrize bis zum Rand mit der Kunstharzmasse gefüllt. Der Rand der Matrize liegt in einer Ebene, so daß die Matrize leicht ohne eine spezielle Befüllungsvorrichtung gefüllt werden kann. Dann wird die Kunstharzmasse mit einer zweiten Folie, der Deckfolie abgedeckt. Da die Kunstharzmasse bis zum oberen Rand der Matrize reicht, bilden sich keine Luftblasen unter der Deckfolie, wobei andererseits toleriert werden kann, daß die Kunstharzmasse etwas über den Rand hinausfließt. Es ist aber günstiger, die Kunstharzmasse vor dem Auflegen der Deckfolie oberflächlich etwas anhärten zu lassen, was beispielsweise durch Bestrahlung oder mit Hilfe von Warmluft geschehen kann.

Anschließend wird die Deckfolie über den gesamten Rand mit der ersten Folie verschweißt. Hierbei kann das Folienmaterial direkt miteinander verschweißt werden, oder auch dadurch, daß vor dem Verschweißen auf den Rand der unteren Folie eine Haftvermittlerschicht aufgebracht wird.

Vorzugsweise werden die Folienränder durch Hochfrequenz miteinander verschweißt, wobei die aus Metall bestehende Matrize die erste Elektrode und eine auf die Deckfolie aufgelegte Metallplatte die zweite Elektrode darstellt.

Dann wird der Einlagekörper entfernt, wobei die erste tiefgezogene Folie gewissermaßen umklappt und sich wieder der Kontur der Matrize anpaßt. In der Kunstharzmasse bildet sich eine Vertiefung, die dem Volumen des entfernten Einlagekörpers entspricht. Eine Verschiebung der Schweißnaht ist nicht möglich, da die Fläche der "Eindellung" der der Kunstharzmasse zugewandten freien Fläche des Einlagekörpers entspricht.

Dann wird die zweite Folie mit Hilfe einer Patrize, deren Form der Form der dem Körper zugewandten schalenförmigen Vertiefung entspricht, dauerhaft den Konturen der Patrize angepaßt. Dies kann dadurch erfolgen, daß man die Deckfolie mit Hilfe der Patrize oder vor dem Auflegen der Patrize mit Hilfe einer gesonderten Wärmequelle auf die Erweichungstemperatur erwärmt. Die zweite Alternative wird vorgezogen, damit die Temperatur der Patrize nicht zwischen der Erweichungstemperatur der Folie und der Aushärtungstemperatur der Kunstharzmasse variiert zu werden braucht, sondern immer bei der Aushärtungstemperatur der Kunstharzmasse gehalten werden kann.

Nach dem Aushärten der Kunstharzmasse erhält man eine Brustprothese mit einem in einer Ebene liegenden Randbereich. Will man die Brustprothese noch weiter verformen, so bringt man die noch nicht ausgehärtete Brustprothese in eine andere Form mit nicht-planem Rand, deren Kontur vorzugsweise der Kontur der Kontur der Körperoberfläche angepaßt ist, und härtet die Kunstharzmasse in dieser Form aus.

Ferner kann man mehrere Schichten von Kunstharzmassen mit unterschiedlichen Eigenschaften, die nach dem Aushärten unterschiedliche Festigkeiten ergeben, einführen, wobei die zweite und gegebenenfalls die folgenden Schichten vorzugsweise dann eingeführt werden, wenn die erste bzw. die zuvor eingefüllte Schicht oberflächlich angehärtet ist. Hierbei kann die erste Schicht so ausgewählt sein, daß sie im ausgehärteten Zustand nach Farbe und/oder Härte einer Brustwarze entspricht. Die folgende Schicht kann dann, wie in der DE-PS 33 36 279 angegeben ist, im ausgehärteten Zustand etwas weicher sein, und beispielsweise aus einer Siliconharzmasse bestehen, die durch Additionsvernetzung ausgehärtet wird. Darüber kann eine Schicht eingefüllt werden, die im ausgehärteten Zustand eine höhere Festigkeit hat. Hierfür kann man beispielsweise ein durch Kondensation vernetzbares Siliconharz verwenden.

Die erfindungsgemäße Brustprothese behält also ihre natürliche Weichheit, die durch die geringere Festigkeit der zweiten Schicht der additionsvernetzten Siliconharzmasse bedingt ist, während sie andererseits aufgrund der höheren Festigkeit der dritten Schicht aus dem kondensationsvernetzten Siliconharz eine gute Formhaltigkeit hat, die es ermöglicht, daß die an den Körper anliegende Fläche der Prothese dauerhaft entsprechend den Körperkonturen gewölbt werden kann, ohne daß Faltenbildungen auftreten, wie es bei einer Brustprothese mit planer Auflagefläche der Fall wäre.

Die erforderliche Formhaltigkeit wird auch dadurch gefördert, daß die etwas festere Siliconharzmasse der dritten Schicht als Bindemittel zwischen den thermoplastischen Folien wirkt, wobei auch die thermoplastischen Folien, insbesondere die erste Folie, zur Erhöhung der Formhaltigkeit beitragen. Die Dicke der dritten Schicht beträgt gewöhnlich mindestens 2 mm, vorzugsweise mindestens 4 mm.

Bezüglich der thermoplastischen Folien bestehen keine besonderen Beschränkungen. Sie müssen lediglich eine gewisse Dehnbarkeit bei Raumtemperatur oder im erwärmten Zustand aufweisen und sich mit der festeren kondensationsvernetzten Siliconharzmasse verbinden. Es können praktisch alle Folien verwendet werden, die für das Tiefzugverfahren geeignet sind. Vorzugsweise sind die Folien jedoch auf Polyurethanbasis aufgebaut.

Bei den additionsvernetzten Harzen reagiert der Vernetzer mit den reaktionsfähigen Stellen der Siliconharzketten, ohne daß, wie bei den kondensationsvernetzten Siliconharzmassen, kleinere Moleküle austreten. Additionsvernetzte und kondensationsvernetzte Siliconkautschukmassen sind an sich bekannt, so daß sie hier nicht näher erläutert zu werden brauchen (vgl. zum Beispiel Chemiker-Zeitung 97 (1973), Seiten 176-180).

Die erforderliche Formhaltigkeit der erfindungsgemäßen Brust prothese ist in erster Linie durch die Zugfestigkeit der ausgehärteten Siliconharzmasse der dritten Schicht bedingt, daneben auch durch die Zugfestigkeit der mit dieser Siliconharzmasse verbundenen thermoplastischen Folien. Die Zugfestigkeit dieser Siliconharzmasse beträgt üblicherweise mindestens 0,05 N/mm², vorzugsweise 0,08 bis 0,15 N/mm². Die ausgehärteten Siliconharzmassen haben gewöhnlich eine Shore-Härte (nach DIN 33505) von etwa 0,2 bis 15, vorzugsweise von etwa 0,1 bis 1 (gemessen mit einem 45%-Plastikkegel, 15 g).

Auf der anderen Seite ist die Zugfestigkeit der ausgehärteten Siliconharzmasse (bzw. -massen) der vom Körper abgewandten Schicht (bzw. Schichten) weit geringer, da diese Schicht(en) die erforderliche Weichheit der Brustprothese bestimmen. Die Zugfestigkeit dieser Siliconharzmassen waren nach der vorstehend genannten DIN-Methode nicht meßbar.

Die erfindungsgemäß verwendeten Siliconharzmassen können mit Hilfe der üblichen Härter ausgehärtet werden, üblicherweise durch Erwärmung. Zu diesem Zweck werden die zur Herstellung der Brustprothesen verwendeten Matrizen und Patrizen gewöhnlich elektrisch beheizt, beispielsweise durch elektrische Widerstandsheizelemente. Eine Erwärmung kann auch mit Hilfe eines heißen fluiden Mediums erfolgen, das durch Heizkanäle in der Matrize und der Patrize strömt.

Zum Aushärten von Siliconharzen werden im allgemeinen Temperaturen von etwa 60 bis 90°C angewendet. Die zur Aushärtung erforderliche Temperatur hängt von verschiedenen Faktoren ab, zum Beispiel von der Art des Kunstharzes und des Vernetzers, von den Mengenverhältnissen dieser Komponenten sowie von der Art und der Konzentration des zur Vernetzung verwendeten Katalysators.

Ein besonderer Vorteil des erfindungsgemäßen Verfahrens besteht darin, daß die Temperatur der Matrizen- und Patrizenformen konstant gehalten werden kann, d.h. eine Abkühlung beim Anheben der tiefgezogenen ersten Folie zum Einsetzen des Einlagekörpers sowie bei der Herausnahme der fertigen Brustprothese ist nicht erforderlich. Dadurch ergibt sich eine beträchtliche Zeit- und Kostenersparnis.

Die erfindungsgemäß verwendeten Kunstharzmassen können verschiedene Zusätze enthalten, beispielsweise Füllstoffe, Farbstoffe, Katalysatoren usw. Vorzugsweise werden die Kunstharzmassen im allgemeinen entsprechend der natürlichen Hautfarbe eingefärbt.

Ferner können in der Kunstharzmasse luftgefüllte Kammern oder Bereiche aus einem anderen Material (z.B. einem Schaumstoff) vorgesehen werden, um das Gewicht und/oder die Festigkeit der Brustprothese zu varieren.

Das erfindungsgemäße Verfahren ist nachstehend anhand der Zeichnung erläutert, wobei Fig. 1 das Tiefziehen der ersten Folie, Fig. 2 das Einlegen des Einlagekörpers und das Verschweißen der Deckfolie mit der ersten Folie und Fig. 3 die Ausbildung der dem Körper zugewandten schalenförmigen Vertiefung zeigt.

Die Matrize 10 ist durch elektrische Heizelemente (nicht dargestellt) zum Beispiel auf 90°C beheizbar. Sie enthält einen Hohlraum, welcher entsprechend der äußeren Form der Brust ausgebildet ist. In den unteren Teil des Hohlraums mündet eine Vakuumleitung 12, die außerhalb der Matrize mit einem Ventil 14 verbunden ist, das das Anlegen eines Vakuums ermöglicht. Der Hohlraum der Matrize ist durch einen planen Rand 16 begrenzt.

Zur Herstellung der Prothese wird zunächst die erste thermoplastische Folie 18 über die erwärmte Matrize 10 gespannt und mit Hilfe des Spannrahmens 20, der durch das Scharnier 22 an der Matrize angelenkt ist, festgeklemmt, indem der Verschluß 24 geschlossen wird. Die Folie 18 erstreckt sich also vom Hohlraum der Matrize bis zum Rand 16 bzw. etwas darüber hinaus.

Nach der Fixierung der Folie 18 wird diese mit Hilfe eines Wärmestrahlers oder mit Warmluft auf die Erweichungstemperatur (bei thermoplastischen Polyurethanfolien etwa 130°C) erwärmt und das Vakuumventil 14 geöffnet, wodurch die Folie 18 tiefgezogen wird und sich an die Wand des Hohlraums der Matrize 10 anlegt, d.h. in eine der äußeren Form der Brust entsprechende Schalenform gebracht wird (in Fig. 1 mit 18a angedeutet). Da die Temperatur der Matrize 10 unterhalb der Erweichungstemperatur der Folie 18 liegt, erstarrt diese und behält ihre Form auch dann bei, wenn das Vakuum durch Umschalten des Ventils 14 aufgehoben wird. Dann wird die tiefgezogene, im Spannrahmen 20 eingeklemmte Folie 18a durch Lösen des Verschlusses 24 aus dem Hohlraum herausgehoben, worauf der Einlagekörper 26 in den Hohlraum gelegt wird. Der Einlagekörper hat das Volumen V₁, das dem Volumen V₂ der dem Körper zugewandten schalenförmigen Vertiefung, deren Herstellung nachstehend noch näher erläutert ist, entspricht. Auch die freie Fläche des Einlagekörpers, d.h. die Fläche, die nicht an der Wand des Hohlraums anliegt (im Schnitt durch die gestrichelte Linie a angedeutet), entspricht der Fläche des Volumens V₂ (in Fig. 3 im Schnitt durch die gestrichelte Linie b angedeutet).

Der Einlagekörper 26 hat in der Mitte eine kleine Vertiefung 28, in die die Kunstharzmasse, die nach der Aushärtung die Brustwarze ergibt, bis zur Höhe h₁ eingefüllt wird.

Nach dem Einsetzen des Einlagekörpers 26 in den Hohlraum (Fig. 2) wird die tiefgezogene Folie 18a mit dem Spannrahmen 20 wieder in die Vertiefung der Matrize 10 eingeführt, wobei sie durch den Einlagekörper in der Mitte nach oben gedrückt und eingedellt wird (durch die Bezugszahl 18b angedeutet), ansonsten aber ihre Form beibehält, da die Temperatur des Einlagekörpers 26 unterhalb der Erweichungstemperatur der Folie liegt. Dann wird das Ventil 14 umgeschaltet und wieder ein Vakuum angelegt, so daß sich die Folie im Bereich 18b dicht an den Einlagekörper anlegt. Dann wird die Kunstharzmasse, die nach dem Aushärten die Brustwarze ergibt, bis zur Höhe h₁ in die Vertiefung 28 eingefüllt und durch Anwendung von Wärmestrahlung oder Warmluft oberflächlich angehärtet.

Anschließend wird die restliche Kunstharzmasse, entweder in einer oder in mehreren Schichten unterschiedlicher Zusammensetzung bis zum Rand 16 eingefüllt. So kann z.B. zunächst eine Schicht aus additionsvernetzbarem Siliconharz bis zur Höhe h₂ und anschließend eine Schicht aus kondensationsvernetzbarem Siliconharz bis zur Höhe h₃, d.h. bis auf die Höhe des Randes 16, eingefüllt werden. Um ein Vermischen der einzelnen Schichten zu verhindern, kann die jeweils untere Schicht durch Wärmestrahlung oder Warmluft oberflächlich angehärtet werden, bevor die nächste Schicht eingefüllt wird.

Dann wird die Deckfolie 30 über die Matrize 10 gespannt, wobei sie am Rand 16 mit der Folie 18 in Berührung kommt.

Anschließend wird eine ebene Metallplatte 32, die als Hochfrequenzschweißelektrode dient, auf die Deckfolie 30 gelegt, worauf diese am Rand 16 mit der Folie 18 verschweißt wird. Der metallische Rand 16 der Matrize 10 dient hierbei als zweite Hochfrequenz-Schweißelektrode.

Nach dem Verschweißen der Folien 18 und 30 wird die Metallplatte 32 entfernt, der Rahmen 20 angehoben und der Einlagekörper 26 aus der Vertiefung entfernt. Der mit 18b bezeichnete Bereich der Folie 18 klappt dann (Fig. 3) in die mit 18a bezeichnete Stellung um, wobei sie die eingefüllte Kunstharzmasse mitnimmt. Gleichzeitig wird die Folie 30 durch Strahlung oder Warmluft erwärmt, wobei sie in die durch 30a angedeutete Stellung abgesenkt wird.

Man erkennt, daß hierbei auf die Folie 18 am Rand 16 kein Zug ausgeübt wird, so daß sich die Schweißnaht nicht verschiebt.

Das Anlegen der Folie im Bereich 18a an die Wand des Hohlraums der Matrize 10 wird durch Anlegen eines Vakuums gefördert. Zuletzt wird die Patrize 34, die wie die Matrize 10 durch Heizelemente (nicht dargestellt) erwärmt ist, aufgelegt. Die Patrize 34 hat eine Auswölbung 34a, deren Volumen dem Volumen der dem Körper zugewandten schalenförmigen Vertiefung und somit auch dem Volumen V₁ bzw. V₂ entsprechen. Die Temperatur der Patrizenauswölbung 34a kann etwas höher sein als die Temperatur der Matrize 10, wenn die Folie 30 zuvor nicht oder nur unvollständig thermoplastisch verformt wurde.

Nach dem Aushärten der Kunstharzmasse aufgrund der Erwärmung durch die Matrize 10 und die Patrize 34 (insbesondere deren Auswölbung 34a) wird das Vakuum aufgehoben, und die ausgehärtete Brustprothese wird durch Anheben des Spannrahmens 20 aus der Vertiefung der Matrize 10 entfernt und nach dem Lösen des Spannrahmens an den Rändern 16 beschnitten.

Wenn man eine Brustprothese wünscht, deren Kontur der Kontur der Körperoberfläche angepaßt ist, wie es in der DE-PS 33 36 279 beschrieben ist, bringt man die noch nicht ausgehärtete Brustprothese nach dem Aufschweißen der Folie 30 auf die Folie 18 in eine andere Matrizenform mit einem entsprechend geformten nicht-planen Rand und legt eine erwärmte Patrize mit einem entsprechend geformten nicht-planen Rand auf, so daß die oberste, dem Körper zugewandte Schicht der Kunstharzmasse erst in dieser anderen Form ausgehärtet wird.

## Patentansprüche

1. Verfahren zur Herstellung einer Brustprothese
(a) aus einer gelartig härtbaren Kunstharzmasse, die zwischen zwei thermoplastischen Folien eingeschlossen ist,
(b) mit einer einseitigen schalenförmigen Vertiefung,
(c) unter Verwendung einer erwärmten, eine Matrize und eine Patrize enthaltenden Form;
welches folgende Schritte umfaßt:
1) die der schalenförmigen Vertiefung abgewandte, gespannte erste Folie (18) wird auf die Matrize (10) gelegt, erwärmt und durch Tiefziehen dauerhaft den Konturen der Matrize (10) angepaßt (Fig. 1);
2) die tiefgezogene erste Folie (18) wird aus der Matrize (10) entfernt;
3) in die Matrize (10) wird ein Einlagekörper (26) eingesetzt, dessen Volumen (V1) und freie Fläche dem Volumen und der Fläche der schalenförmigen Vertiefung (V2) entsprechen;
4) die tiefgezogene erste Folie (18) wird an den Einlagekörper (26) in der Matrize (10) angelegt (Fig. 2);
5) die Matrize (10) wird bis zu ihrem Rand mit der Kunstharzmasse gefüllt;
6) die Kunstharzmasse wird mit einer zweiten Folie (30) abgedeckt und über deren gesamten Rand (16) mit der ersten Folie (18) verschweißt;
7) der Einlagekörper (26) wird entfernt;
8) die zweite Folie (30) wird mit Hilfe der Patrize (34a), deren Form der Form der schalenförmigen Vertiefung (V2) entspricht, dauerhaft den Konturen der Patrize (34a) anpaßt (Fig. 3);
9) die Kunstharzmasse wird ausgehärtet.

2. Verfahren nach Anspruch 1, dadurch gekennzeichnet, daß man die erste Folie (18) vor dem Tiefziehen durch Erwärmen von außen auf die Erweichungstemperatur erhitzt, wogegen man die Matrize (10) auf einer Temperatur zwischen der Erweichungstemperatur der ersten Folie (18) und der Aushärtungstemperatur der Kunstharzmasse hält.

3. Verfahren nach Anspruch 1 oder 2, dadurch gekennzeichnet, daß man den Einlagekörper (26) mit einer Temperatur einsetzt, die unterhalb der Erweichungstemperatur der ersten Folie liegt.

4. Verfahren nach einem der Ansprüche 1 bis 3, dadurch gekennzeichnet, daß man die Folienränder (16) durch Hochfrequenz miteinander verschweißt, wobei die Matrize (10) die erste Elektrode und eine auf die zweite Folie (30) aufgelegte Metallplatte (32) die zweite Elektrode darstellt.

5. Verfahren nach einem der Ansprüche 1 bis 4, dadurch gekennzeichnet, daß man die zweite Folie (30) mit Hilfe der Patrize (34a) oder vor dem Auflegen der Patrize (34a) mit Hilfe einer gesonderten Wärmequelle auf die Erweichungstemperatur erwärmt.

6. Verfahren nach einem der Ansprüche 1 bis 5, dadurch gekennzeichnet, daß man die zweite Folie (30) auflegt, nachdem die in der Matrize (10) befindliche Kunstharzmasse oberflächlich angehärtet ist.

7. Verfahren nach einem der Ansprüche 1 bis 6, dadurch gekennzeichnet, daß man die noch nicht ausgehärtete Brustprothese in eine andere Form mit nicht-planem Rand bringt, deren Kontur der Kontur der menschlichen Körperoberfläche angepaßt ist, und die Kunstharzmasse in dieser Form aushärtet.

8. Verfahren nach einem der Ansprüche 1 bis 7, dadurch gekennzeichnet, daß man mehrere Schichten von Kunstharzmasse mit unterschiedlichen Eigenschaften einführt, wobei die zweite und gegebenenfalls die folgenden Schichten dann eingefüllt werden, wenn die erste bzw. die zuvor eingefüllte Schicht oberflächlich angehärtet ist.

9. Verfahren nach Anspruch 8, dadurch gekennzeichnet, daß die erste Schicht so ausgewählt ist, daß sie im ausgehärteten Zustand nach Farbe und/oder Härte einer Brustwarze entspricht.

10. Verfahren nach einem der Ansprüche 1 bis 9, dadurch gekennzeichnet, daß man in der Kunstharzmasse luftgefüllte Kammern oder Bereiche aus einem anderen Material vorsieht.

## Claims

1. Process for producing a breast prosthesis
(a) from a synthetic resin composition that can be cured to a gelatinous consistency and that is enclosed between two thermoplastic films,
(b) with a one-sided cup-shaped recess,
(c) using a heated mold including a female die and a male die;
which (process) comprises the following steps:
1) the first tensioned film (18) facing away from the cup-shaped recess is placed on the female die (10), is heated and is permanently conformed to the contours of the female die (10) by deep-drawing (Fig. 1);
2) the deep-drawn first film (18) is removed from the female die (10);
3) an insert (26) is placed in the female die (10), the volume (V1) and the free area of which correspond to the volume and the area of the cup-shaped recess (V2);
4) the deep-drawn first film (18) is placed over the insert (26) in the female die (10) (Fig. 2);
5) the female die (10) is filled to its upper edge with a synthetic resin composition;
6) the synthetic resin composition is covered with a second film (30) and is welded over its entire edge (16) with the first film (18);
7) the insert (26) is removed;
8) the second film (30), by means of the male die (34a) whose form corresponds to the form of the cup-shaped recess (V2) is permanently adapted to the contours of the male die (34a) (Fig. 3);
9) the sythetic resin composition is cured.

2. Process according to claim 1, characterized in that the first film (18) is externally heated to a softening temperature before the deep drawing step, whereas the female die (10) is maintained at a temperature between the softening temperature of the first film (18) and the curing temperature of the synthetic resin composition.

3. Process according to claims 1 or 2, characterized in that the insert (26) is placed at a temperature below the softening temperature of the first film.

4. Process according to anyone of claims 1 ot 3, characterized in that the film edges (16) are welded by high frequency, wherein the female die (10) forms the first electrode and a metal plate (32) placed over the second film (30) forms the second electrode.

5. Process according to anyone of claims 1 to 4, characterized in that the second film (30) is heated to the softening temperature with the male die (34a), or prior to placing the male die (34a), by means of a separate heat source.

6. Process according to anyone of claims 1 to 5, characterized in that the second film (30) is placed after the synthetic resin composition in the female die (10) has been surface-cured.

7. Process according to anyone of claims 1 to 6, characterized in that the incompletely cured breast prosthesis is placed in another mold with a non-planar edge whose contour corresponds to the contour of the human body surface, and the synthetic resin composition is cured in this mold.

8. Process according to anyone of claims 1 to 7, characterized in that several layers of the synthetic resin composition having different properties are inserted, the second and optionally the following layers are filled in, after the first and the previously filled-in layer, respectively, have been surface-cured.

9. Process according to claim 8, characterized in that the first layer is selected so that after curing, it corresponds in color and/or hardness to the nipple of a breast.

10. Process according to anyone of claims 1 to 9, characterized in that air-filled chambers or regions of another material are provided in the synthetic resin composition.

## Revendications

1. Procédé pour la fabrication d'une prothèse mammaire
(a) composée d'une masse de résine synthétique durcissable sous forme de gel, qui est enfermée entre deux feuilles thermoplastiques,
(b) avec une dépression en forme de coupe unilatérale,
(c) utilisant un moule chauffé contenant un moule négatif et un moule positif,
caractérisé en ce qu'il comprend les étapes suivantes :
1) la première feuille (18) opposée à la dépression en forme de coupe, tendue, est posée sur le moule négatif (10), chauffée et adaptée de manière permanente par thermoformage aux contours du moule négatif (10) (Figure 1) ;
2) la première feuille (18) thermoformée est retirée du moule négatif (10) ;
3) un corps d'insert (26) dont le volume (V1) et la surface libre correspondent au volume et à la surface de la dépression en forme de coupe (V2) est disposé dans le moule négatif (10) ;
4) la première feuille (18) thermoformée est posée sur le corps d'insert (26) dans le moule négatif (10) (Figure 2) ;
5) le moule négatif (10) est rempli jusqu'au bord de la masse de résine synthétique ;
6) la masse de résine synthétique est recouverte d'une seconde feuille (30) et soudée avec la première feuille (18) sur tout le bord (16) de celle-ci ;
7) le corps d'insert (26) est enlevé ;
8) la seconde feuille (30) est adaptée de manière permanente aux contours du moule positif (34a) à l'aide du moule positif (34a), dont la forme correspond à la forme de la dépression en forme de coupe (V2);
9) la masse de résine est durcie.

2. Procédé selon la revendication 1, caractérisé en ce que l'on chauffe la première feuille (18) avant le thermoformage par échauffement de l'extérieur jusqu'à la température de ramollissement, alors que l'on maintient le moule négatif (10) à une température comprise entre la température de ramollissement de la première feuille (18) et la température de durcissement de la masse de résine synthétique.

3. Procédé selon la revendication 1 ou 2, caractérisé en ce que l'on introduit le corps d'insert (26) à une température inférieure à la température de ramollissement de la première feuille.

4. Procédé selon l'une ou l'ensemble des revendications 1 à 3, caractérisé en ce que l'on soude les bords (16) des feuilles entre eux par soudage à haute fréquence, le moule négatif (10) constituant la première électrode et une plaque métallique (32) posée sur la seconde feuille (30), la seconde électrode.

5. Procédé selon l'une ou l'ensemble des revendications 1 à 4, caractérisé en ce que l'on chauffe la seconde feuille (30) à la température de ramollissement à l'aide du moule positif (34a) ou à l'aide d'une source de chaleur séparée avant l'application du moule positif (34a).

6. Procédé selon l'une ou l'ensemble des revendications 1 à 5, caractérisé en ce que l'on pose la seconde feuille (30) après que la masse de résine synthétique qui se trouve dans le moule négatif (10) a durci en surface.

7. Procédé selon l'une ou l'ensemble des revendications 1 à 6, caractérisé en ce que l'on donne à la prothèse mammaire non encore durcie une autre forme à bord non plan, dont le contour est adapté au contour de la surface du corps humain, et en ce que la masse de résine synthétique durcit sous cette forme.

8. Procédé selon l'une ou l'ensemble des revendications 1 à 7, caractérisé en ce que l'on introduit plusieurs couches de masse de résine synthétique ayant des propriétés différentes, la seconde couche et éventuellement les couches suivantes étant versées lorsque la première couche ou la couche versée précédemment est durcie en surface.

9. Procédé selon la revendication 8, caractérisé en ce que la première couche est choisie de telle sorte que sa couleur et/ou sa consistance à l'état durci correspondent à celles d'un mamelon.

10. Procédé selon l'une ou l'ensemble des revendications 1 à 9, caractérisé en ce que l'on prévoit dans la masse de résine synthétique des cavités emplies d'air ou des zones faites d'un autre matériau.
